(19) Europäisches Patentamt
European Patent Office
Office européen des brevets

(11) EP 3 381 440 A2

# (12) EUROPEAN PATENT APPLICATION

(43) Date of publication:
**03.10.2018 Bulletin 2018/40**

(21) Application number: **18165449.2**

(22) Date of filing: **03.04.2018**

(51) Int Cl.:
***A61K 8/73*** *(2006.01)*
***A61K 8/67*** *(2006.01)*
***A61Q 19/00*** *(2006.01)*
***A61P 17/10*** *(2006.01)*
***A61Q 19/08*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2017 US 201715475516**

(71) Applicant: **JOHNSON & JOHNSON CONSUMER INC.**
**Skillman, NJ 08558 (US)**

(72) Inventors:
- **FEVOLA, Michael James**
  **Belle Mead, NJ 08502 (US)**
- **KAUR, Simarna**
  **Skillman, NJ New Jersey 08558 (US)**
- **SOUTHALL, Michael D.**
  **Skillman, NJ New Jersey 08558 (US)**
- **FASSIH, Ali**
  **Skillman, NJ New Jersey 08558 (US)**

(74) Representative: **Carpmaels & Ransford LLP**
**One Southampton Row**
**London WC1B 5HA (GB)**

## (54) METHODS OF IMPROVING THE ACTIVITY OF RETINOIDS

(57) Methods of treating skin in need of treatment with retinoids are provided, which comprise cleansing the skin with a cleansing composition comprising a low irritation polymeric cleansing agent prior to treating with a retinoid. This improves the activity of the retinoid for treating for example signs of skin aging, acne, or rosacea.

EP 3 381 440 A2

## Description

### Field of the Invention

The present invention relates to methods of treating skin in need of treatment with retinoids, which comprise cleansing the skin with a cleansing composition comprising a low irritation polymeric cleansing agent prior to treating with the retinoids. These methods improve the activity of the retinoids and can enable superior outcomes.

### Background of the Invention

Retinoids have been used in a variety of prescription and cosmetic topical compositions for treating skin conditions such as signs of skin aging, acne, and rosacea.

For example, tretinoin is used in prescription acne products as well as prescription anti-wrinkle products such as RENOVA® commercially available from Obagi Medical Products, Inc. Retinol is used in cosmetics for example NEUTROGENA® Rapid Wrinkle Repair® commercially available from Johnson & Johnson Consumer Inc. NEUTROGENA® Rapid Wrinkle Repair® is used to fade the look of wrinkles in skin, smooth fine lines, improve skin texture, and brighten skin tone. Retinol in particular has proven to be a highly efficacious and cost effective cosmetic ingredient, and improvements in its activity and delivery into the skin are always desirable.

Applicants have now discovered that pretreatment of skin by cleansing with a cleansing composition comprising particular low irritation polymeric cleansing agents (LIPCAs) before topical application of a retinoid surprisingly increases the activity of the retinoid.

Multi-step skin care regimens are known. For example, WO 2010/085753 discloses a treatment regimen including cleansing at least a portion of an area of skin afflicted with rosacea with a cleanser; applying a composition comprising metronidazole to at least a portion of the afflicted area, and applying an anti-redness composition to at least a portion of the cleansed and metronidazole-treated area. Use of "gentle" skin cleansers CETAPHIL® (Galderma Laboratories) and NU-DERM® (Obagi Medical Products, Inc.) are disclosed. In addition, optional application of compositions comprising retinoids and other ingredients, are disclosed.

The directions on the NEUTROGENA® Rapid Wrinkle Repair® package also state that the product should be applied to cleansed skin, although no particular cleanser is indicated.

It may also be noted that a cleansing composition comprising a LIPCA, potassium acrylates copolymer, is commercially available from Johnson & Johnson Consumer Inc. as NEUTROGENA® Ultra Gentle Daily Cleanser.

### Summary of the Invention

The invention provides a method for treating skin, comprising in sequence: (a) cleansing skin in need of treatment for signs of skin aging, acne, or rosacea with a cleansing composition comprising a superhydrophilic amphiphilic copolymer (SAC); and (b) topically applying to the skin a leave-on composition comprising a retinoid.

The invention also provides a non-therapeutic method for treating skin, comprising in sequence: (a) cleansing skin in need of treatment for signs of skin aging, acne, or rosacea with a cleansing composition comprising a superhydrophilic amphiphilic copolymer (SAC); and (b) topically applying to the skin a leave-on composition comprising a retinoid.

The invention also provides a leave-on composition comprising a retinoid for use in a method for treating skin showing signs of aging, acne and/or rosacea wherein the method comprises:

(a) cleansing skin in need of treatment with a cleansing composition comprising a superhydrophilic amphiphilic copolymer; and

(b) topically applying to the skin the leave-on composition comprising a retinoid.

The invention also provides a kit comprising: (a) a cleansing composition comprising a SAC; and (b) a leave-on composition comprising a retinoid.

The invention also provides a kit for use in a method for treating skin showing signs of skin aging, acne, and/or rosacea wherein the kit comprises: (a) a cleansing composition comprising a SAC; and (b) a leave-on composition comprising a retinoid.

The invention further provides a method of increasing the activity of a retinoid, comprising cleansing skin in need of treatment with retinoids with a cleansing composition comprising a SAC prior to contacting the skin with the retinoid.

The invention further provides a retinoid for use in a method for treating skin in need of treatment with retinoids, wherein the method comprises cleansing skin in need of treatment with a cleansing composition comprising a SAC prior

to contacting the skin with the retinoid, wherein the method increases the activity of the retinoid.

The invention also provides a method for treating skin, comprising in sequence: (a) cleansing skin in need of treatment for signs of skin aging, acne, or rosacea with a cleansing composition comprising a low molecular weight hydrophobically modified polymer (HMP); and (b) topically applying to the skin a leave-on composition comprising a retinoid.

The invention also provides a non-therapeutic method for treating skin, comprising in sequence: (a) cleansing skin in need of treatment for signs of skin aging, acne, or rosacea with a cleansing composition comprising a low molecular weight hydrophobically modified polymer (HMP); and (b) topically applying to the skin a leave-on composition comprising a retinoid.

The invention also provides a leave-on composition comprising a retinoid for use in a method for treating skin showing signs of aging, acne and/or rosacea wherein the method comprises:

(a) cleansing skin in need of treatment with a cleansing composition comprising a low molecular weight hydrophobically modified polymer; and
(b) topically applying to the skin the leave-on composition comprising a retinoid.

The invention also provides a kit comprising: (a) a cleansing composition comprising a low molecular weight HMP; and (b) a leave-on composition comprising a retinoid.

The invention also provides a kit for use in a method for treating skin showing signs of skin aging, acne, and/or rosacea wherein the kit comprises: (a) a cleansing composition comprising a low molecular weight HMP; and (b) a leave-on composition comprising a retinoid.

The invention further provides a method of increasing the activity of a retinoid, comprising cleansing skin in need of treatment with retinoids with a cleansing composition comprising a low molecular weight HMP prior to contacting the skin with the retinoid.The invention further provides a retinoid for use in a method for treating skin in need of treatment with retinoids, wherein the method comprises cleansing skin in need of treatment with a cleansing composition comprising a low molecular weight HMP prior to contacting the skin with the retinoid, wherein the method increases the activity of the retinoid.

**Detailed Description of the Invention**

Unless defined otherwise, all technical and scientific terms used herein have the same meaning as commonly understood by one of ordinary skill in the art to which the invention belongs. All publications, patent applications, patents, and other references mentioned herein are incorporated by reference.

As used herein, "topically applying" means directly laying on or spreading on outer skin, the scalp, or hair, e.g., by use of the hands or an applicator such as a wipe, roller, or spray.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "cosmetically effective amount" means an amount of a physiologically active compound or composition sufficient for treating one or more signs of skin aging, but low enough to avoid serious side effects. The cosmetically effective amount of the compound or composition will vary with the particular condition being treated, the age and physical condition of the end user, the severity of the condition being treated/prevented, the duration of the treatment, the nature of other treatments, the specific compound or product/composition employed, the particular cosmetically-acceptable carrier utilized, and like factors.

As used herein, "cosmetically acceptable" means that the ingredients the term describes are suitable for use in contact with tissues (e.g., the skin or hair) without undue toxicity, incompatibility, instability, irritation, allergic response, or the like.

As used herein, a "cosmetically acceptable active agent" is a compound (synthetic or natural) that has a cosmetic or therapeutic effect on the skin or hair.

As used herein, "treating" refers to mitigating, reducing, preventing, improving, or eliminating the presence or signs of a condition or disorder.

The present invention is suitable for treating signs of skin aging. As used herein, "signs of skin aging" includes the presence of lines and wrinkles, loss of elasticity, uneven skin, and blotchiness. Preferably, the sign of aging is the presence of lines and wrinkles and/or loss of elasticity.

As used herein, "wrinkle" includes fine lines, fine wrinkles, or coarse wrinkles. Examples of wrinkles include, but are not limited to, fine lines around the eyes (e.g., "crow's feet"), forehead and cheek wrinkles, frown-lines, and laugh-lines around the mouth.

As used herein, "loss of elasticity" includes loss of elasticity or structural integrity of the skin or tissue, including

but not limited to sagging, lax and loose tissue. The loss of elasticity or tissue structure integrity may be a result of a number of factors, including but not limited to disease, aging, hormonal changes, mechanical trauma, environmental damage, or the result of an application of products, such as a cosmetics or pharmaceuticals, to the tissue.

As used herein, "uneven skin" means a condition of the skin associated with diffuse or mottled pigmentation, which may be classified as hyperpigmentation, such as post-inflammatory hyperpigmentation.

As used herein, "blotchiness" means a condition of the skin associated with redness or erythema.

The invention is also suitable for treating acne. As used herein, "acne" refers to disorders resulting from the actions of hormones and other substances on the sebaceous glands and hair follicles, typically leading to clogged pores and the formation of inflammatory or non-inflammatory lesions on the skin. Specifically, it relates to blemishes, lesions, or pimples, pre-emergent pimples, blackheads, and/or whiteheads. As used herein, a "pre-emergent pimple" is an inflamed follicle that are not visually apparent on the surface of the skin with the naked eye (e.g., as a lesion).

The invention is also suitable for treating rosacea. As used herein, "rosacea" means skin with persistent erythema with or without papules, pustules, or nodules.

Unless otherwise indicated, a percentage or concentration refers to a percentage or concentration by weight (i.e., % (W/W). Unless stated otherwise, all ranges are inclusive of the endpoints, e.g., "from 4 to 9" includes the endpoints 4 and 9.

Where applicable, chemicals are specified according to International Nomenclature of Cosmetic Ingredient (INCI) names. Additional information, including suppliers and trade names, can be found under the appropriate INCI monograph in the International Cosmetic Ingredient Dictionary and Handbook, 16th Edition published by the Personal Care Products Council, Washington DC, or via the Personal Care Products Council's On-Line INFOBASE, http://online.personalcarecouncil.org/jsp/Home.jsp.

Cleansing Composition

According to the invention, prior to treating skin with a retinoid, the skin is cleansed with a cleansing composition comprising a low irritation polymeric cleansing agent (LIPCA).

The LIPCA may comprise at least one superhydrophilic amphiphilic copolymer (SAC).

Suitable SACs, and suitable cleansing compositions for use in the invention containing them, are described for example in US 8,258,250, the disclosure of which is incorporated by reference herein.

As used herein, the term "superhydrophilic amphiphilic copolymer," ("SAC") may be defined as a copolymer that may be represented by the following general structure:

$$-\left[\mathrm{SRU}\right]_{s}-\left[\mathrm{ARU}\right]_{a}-\left[\mathrm{HRU}\right]_{h}-$$

Wherein an "SRU" is a superhydrophilic repeat unit as defined herein, an "ARU" is an amphiphilic repeat unit as defined herein, an "HRU" is a hydrophilic repeat unit as defined herein, wherein $s \geq 2$, $a > 0$, $h \geq 0$, and the total number of repeat units, s+a+h is between 4 and about 1000. The term "between," when used herein to specify a range such as "between 4 and about 1000," is inclusive of the endpoints, e.g. "4" and "about 1000." The total number of repeat units in the SAC is based on the weight-average molecular weight ($M_w$) of the SAC; thus the number of repeat units, as discussed herein are "weight average" as well. As will be recognized by one of skill in the art, the pattern of repeat units (SRUs, ARUs, HRUs) incorporated in SACs of the present invention are generally random; however, they may also have alternating, statistical, or blocky incorporation patterns. In addition, SAC architectures may be linear, star-shaped, branched, hyperbranched, dendritic, or the like.

As used herein, the term "superhydrophilic repeat unit," ("SRU") may be defined as a repeat unit that comprises two or more hydrophilic moieties and no hydrophobic moieties.

As used herein, the term "amphiphilic repeat unit," ("ARU") may be defined as a repeat unit that comprises at least one hydrophilic moiety and at least one hydrophobic moiety.

As will be readily understood by those of skill in the art, the term "hydrophilic repeat unit," ("HRU") may be defined as a repeat unit that comprises one and only one hydrophilic moiety and no hydrophobic moieties. Synthetic routes for achieving the SACs of the present invention include via post-polymerization modification of precursor polymers comprising superhydrophilic repeat units to render some repeat units amphiphilic. Nonlimiting examples include the reaction of superhydrophilic polymers comprised of repeat units comprising multiple hydroxyl functionalities, for example, starch, hydroxyethylcellulose, dextran, inulin, pullulan, poly(glyceryl methacrylate), poly[tris(hydroxymethyl)acrylamidomethane)], or poly(sucrose methacrylate), with reagents that will result in amphiphilic repeat units. Examples of suitable reaction schemes include:

i) esterification with alkenyl succinic anhydrides,

ii) etherification with 1,2-epoxyalkanes,

iii) etherification of with 3-chloro-2-hydroxypropylalkyldimethylammonium chlorides, and

iv) esterification with monoalkyl phosphate esters.

Preferably, the SAC for use in the present invention is a polymer having multiple hydroxyl functionalities that is post-polymerization modified to convert some of the repeat units to amphophilic repeat units. Preferably, the polymer, e.g., a starch such as a starch dextrin polymer, is esterified with an alkenyl succinic anhydride to convert some of the superhydrophilic anhydroglucose units to ARUs. The structure of one suitable resulting SAC may be the C-6 sodium dextrin alkenylsuccinate, represented below:

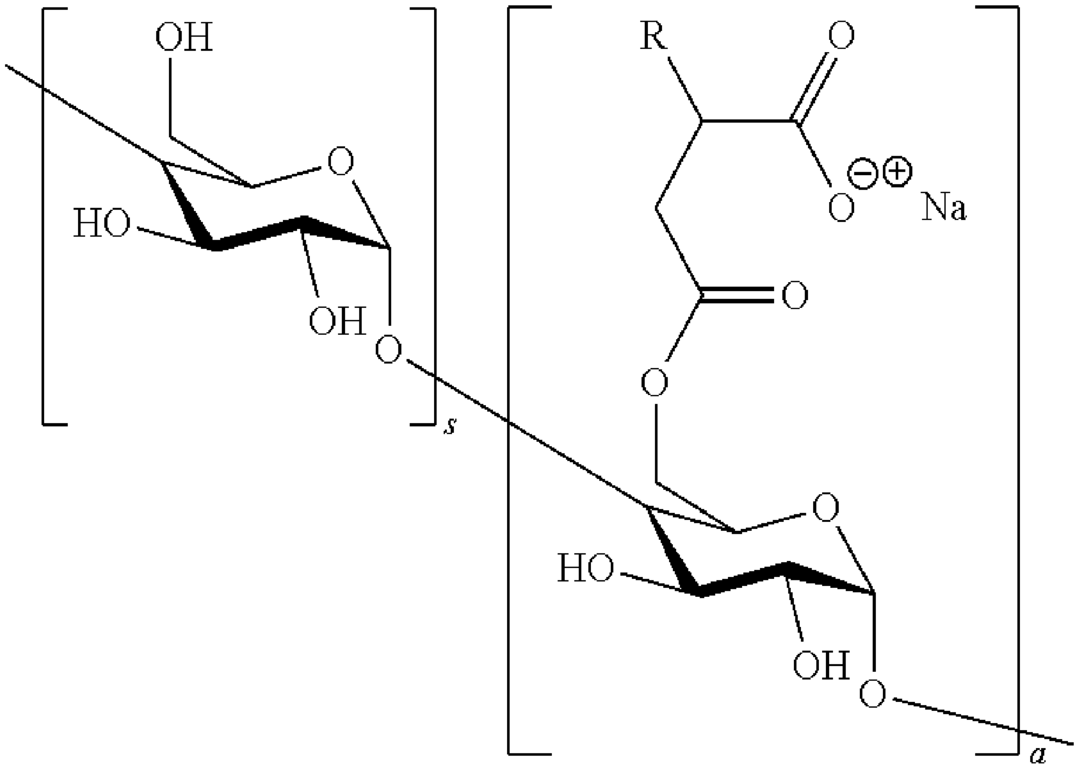

For example, the SAC may be a sodium dextrin dodecenylsuccinate, if R = $C_{12}H_{23}$. As will be recognized by one skilled in the art, such alkenyl succinate esters of polysaccharides may be synthesized as described, for example, in U.S. Pat. No. 2,661,349 incorporated herein by reference. Depending on the nature of the reaction conditions, molecular architecture, type of sugar repeat units, branch points and molecular weight, the modification of the sugar repeat units (anhydroglucose units) may also occur at the C-2, C-3 or C-4 positions in addition to the C-6 position shown above.

The SACs derived from the reaction of the starting polysaccharide with the hydrophobic reagent comprise a polysaccharide bound with the hydrophobic reagent. Preferably, the SAC is a starch-based polysaccharide modified with one or more hydrophobic reagents.

Examples of suitable starches include those derived from such plants as corn, wheat, rice, tapioca, potato, sago, and the like. Such starches can be of a native variety or those developed by plant breeding or by gene manipulation. In the composition of the invention, the starches may include either the waxy versions of such starches (containing less than 5% amylose), high amylose starches (containing more than 40% amylose), those with a modified chain length (such as those disclosed in U.S. Pat. No. 5,954,883, the disclosure of which is incorporated by reference herein), and/or combinations thereof.

Preferably, the starting starch is potato starch or tapioca starch. Preferably, the starting starch is a waxy potato starch or waxy tapioca starch. The starch-based polysaccharide may be modified by dissolving such low molecular weight starch or "dextrin" in water and reacting such starch with a hydrophobic reagent. The starch is desirably processed to lower its molecular weight by techniques known in the art, e.g., action of acid and heat, enzymatic, or thermal processing.

The viscosity of the aqueous solution of the polymeric surfactant is desirably low to minimize the detrimental effect of a high solids level of surfactant with pumping or flow of the solution. For this reason, in the composition of the invention, the Brookfield viscosity measured at room temperature (about 23° C) at 200 rpm using spindle #3 for the polymeric surfactants of this invention may be less than about 1000 cps at 10% aqueous solids based on the total weight of the solution. The Brookfield viscosity measured at room temperature (about 23° C) at 200 rpm using spindle #3 of the 10% aqueous solution may be less than about 25 cps. The Brookfield viscosity measured at room temperature (about 23° C) at 200 rpm using spindle #3 of a 10% aqueous solution may be less than about 10 cps.

Preferably, the starch-based polysaccharide is modified with alkenyl succinic anhydride. Surprisingly, it has been found that a substituted succinic anhydride containing a $C_{12}$ or longer side chain provides improved foam volume and foam stability than substituted succinic anhydrides having less than a $C_{12}$ side chain. Preferably, the alkenyl succinic anhydride is dodecenylsuccinic anhydride (DDSA). Exemplary treatment levels of the DDSA, on the dry basis of low molecular weight ranges from about 3 to about 25%. The treatment level may be from about 5 to about 15% DDSA based on the dry weight of low molecular weight starting starch.

In the composition of the invention, the SAC may be derived from the reaction of the starting polysaccharide and DDSA, and the bound DDSA on the starch-based polysaccharide may be of from about 3 about 15% based on the weight of dry starch. The bound DDSA may be between 5 and 12% based on the dry weight of starch.

In the composition of the invention, the hydrophobic reagent may be a highly branched version of DDSA containing a 12 carbon side chain made from tetramerization of propene. It has been found that when the tetrapropene is then reacted with maleic anhydride in an ene-type reaction, it forms highly branched tetrapropenyl succinic anhydride (TPSA). Because this material is a slightly viscose oil and has acceptable water solubility (e.g., at about 2-5% in water at 23° C), this reagent is capable of reacting favorably with the low molecular weight polysaccharide. In the composition of this invention, therefore, the hydrophobic reagent used to modify the low molecular weight starch may be TPSA.

Preferably, the starch-based polysaccharide is modified with a long chain quaternary compound having at least one chain containing 3 or more carbon atoms. The long chain quaternary compound may have at least one chain containing 6 or more and more preferably 12 or more carbon atoms, such as 3-chloro-2-hydroxypropyl-dimethyldodecylammonium chloride (sold commercially as QUAB® 342 by QUAB Chemicals) or the epoxide form of such compound, 2,3 epoxypropyldimethyldodecylammonium chloride.

Preferably, in the composition of the invention, the SAC is a starch-based polysaccharide derived from potato or tapioca modified with dodecenyl succinic anhydride, wherein the SAC has a mole percent of amphiphilic units that is at least 5 but less than 10% and a weight average molecular weight that is less than about 200,000.

In addition to starch-based polysaccharides, other polysaccharides are suitable for use in the SAC. Such polysaccharides may be derived from plant sources and those based on sugar-type repeat units. Some non-limiting examples of these polysaccharides are guar, xanthan, pectin, carrageenan, locust bean gum, and cellulose, including physical and chemically modified derivatives of the above. Physical, chemical and enzymatic degradation of these materials may be necessary to reduce the molecular weight to the desired range to provide the viscosity for the desired application. Chemical modification can also be performed to provide additional functional properties (e.g., cationic, anionic or non-ionic) such as treatment with propylene oxide (PO), ethylene oxide (EO), alkyl chlorides (alkylation) and esterification such as 3-chloro-2-hydroxypropyl-trimethylammonium chloride, sodium tripolyphosphate, chloroacetic acid, epichlorohydrin, phosphorous oxychloride and the like.

The LIPCA may comprise at least one low molecular weight hydrophobically modified polymer (HMP).

Suitable low molecular weight HMPs, and suitable cleansing compositions for use in the invention containing them, are described for example in US 8,025,902, the disclosure of which is incorporated by reference herein.

Examples of low molecular weight HMPs include low-molecular weight acrylic, other ethylenically-unsaturated polymers, polyesters, polycarbonates, polyanhydrides, polyamides, polyurethanes, polyureas, polyimides, polysulfones, polysulfides, combinations of two or more thereof, and the like.

Examples of suitable low molecular weight acrylic polymers include hydrophobically-modified acrylic, polysaccharide, cellulose, starch polymers, combinations of two or more thereof, and the like. Suitable low molecular weight acrylic polymers include hydrophobically-modified acrylic polymers, as well as other acrylic polymers, any of which may be formed via solution, suspension, precipitation, dispersion, emulsion, inverse emulsion, microemulsion, micellar polymerization methods, and combinations of two or more thereof. The acrylic polymers for use in the present invention may be derived from any one or more monomers selected from the group consisting of (meth)acrylates, (meth)acrylamides, vinyl ethers, esters, and amides, allyl ethers, esters, amines, and amides, itaconates, crotonates, styrenics, and olefins. The acrylic polymers may be comprised of monomers that are nonionic hydrophilic, nonionic hydrophobic, anionic, cationic, zwitterionic, nonassociative macromeric, associative macromeric, or multifunctional/crosslinking in nature.

As used herein the term "low molecular weight" polymer refers to a polymer having a number average molecular weight ($M_n$) of about 100,000 or less as measured by gel permeation chromatography (GPC) calibrated with a poly(methyl methacrylate) (PMMA) standard. (As used herein, unless otherwise specified, all number average molecular weights

($M_n$) refer to molecular weight measured by gel permeation chromatography (GPC) calibrated with a poly(methyl methacrylate) (PMMA) standard.) Preferably, low-molecular weight polymers are those having molecular weight ranges of from about 5,000 to about 80,000 $M_n$, more preferably from about 10,000 to about 50,000 $M_n$, and more preferably between about 15,000 and 40,000 $M_n$.

Certain HMPs and methods of making such polymers are described in U.S. Patent No. 6,433,061, issued to Marchant et al., the disclosure of which is incorporated herein by reference. The polymeric materials useful in this invention are preferably non-crosslinked, linear acrylic copolymers that are very mild to the skin and mucosa. These non-crosslinked, linear polymers are preferably of low molecular weight having a $M_n$ of 100,000 or less. The copolymeric compound is polymerized from at least two monomeric components. The first monomeric component is selected from one or more $\alpha,\beta$-ethylenically unsaturated monomers containing at least one carboxylic acid group. This acid group can be derived from monoacids or diacids, anhydrides of dicarboxylic acids, monoesters of diacids, and salts thereof. The second monomeric component is hydrophobically modified (relative to the first monomeric component) and is selected from one or more $\alpha,\beta$-ethylenically unsaturated non-acid monomers containing a $C_1$ to $C_9$ alkyl group, including linear and branched $C_1$ to $C_9$ alkyl esters of (meth)acrylic acid, vinyl esters of linear and branched $C_1$ to $C_{10}$ carboxylic acids, and mixtures thereof. In one aspect of the invention, the second monomeric component is represented by the formula:

$$CH_2{=}CRX$$

wherein R is hydrogen or methyl; X is $-C(O)OR_1$ or $-OC(O)R_2$; $R_1$ is linear or branched $C_1$ to $C_9$ alkyl; and $R_2$ is hydrogen or linear or branched $C_1$ to $C_9$ alkyl. In another aspect of the invention $R_1$ and $R_2$ is linear or branched $C_1$ to $C_8$ alkyl and in a further aspect $R_1$ and $R_2$ are linear or branched $C_2$ to $C_5$ alkyl.

Preferably the hydrophobically modified polymers comprise, consist essentially of, or consist of a low molecular weight, non-crosslinked, linear acrylic copolymer derived from at least one first monomeric component selected from the group consisting of (meth)acrylic acid and at least one second monomeric component selected from the group consisting of one or more $C_1$ to $C_9$ alkyl (meth)acrylates, wherein the low molecular weight copolymer has a number average molecular weight of about 100,000 or less.

Exemplary first monomeric components include (meth)acrylic acid, itaconic acid, citraconic acid, maleic acid, fumaric acid, crotonic acid, aconitic acid, and mixtures thereof. Exemplary second monomeric components include ethyl (meth)acrylate, butyl (meth)acrylate, 2-ethylhexyl (meth)acrylate, vinyl formate, vinyl acetate, 1-methylvinyl acetate, vinyl propionate, vinyl butyrate, vinyl 2-ethylhexanoate, vinyl pivalate, vinyl neodecanoate, and mixtures thereof.

As used herein, the terms "(meth)acrylic" acid and "(meth)acrylate" are meant to include the corresponding methyl derivatives of acrylic acid and the corresponding alkyl acrylate. For example, "(meth)acrylic" acid refers to acrylic acid and/or methacrylic acid and "(meth)acrylate" refers to alkyl acrylate and/or alkyl methacrylate.

More preferably, said first monomeric component is selected from the group consisting of (meth)acrylic acid and said second monomeric component is selected from the group consisting of at least one $C_1$ to $C_9$ alkyl (meth)acrylate.

The non-crosslinked, linear acrylic copolymer compounds can be synthesized via free radical polymerization techniques known in the art. In one aspect of the invention, the weight ratio of the first monomeric component to the second monomeric component utilized ranges from about 20:80 to about 50:50. In another aspect the weight ratio of the first monomeric component to the second monomeric component is about 35:65, and in a further aspect the weight ratio of first monomeric component to second monomeric component is about 25:75.

Methods of synthesizing the polymers useful in the compositions and methods of this invention may be found in U.S. Patent No. 6,433,061 which is hereby incorporated herein by reference.

The linear copolymeric materials useful in the methods and compositions of this invention preferably have a viscosity of 500 mPa·s or less (Brookfield RVT, 20 rpm, spindle no. 1) at a 5 wt. % polymer solids concentration in deionized water and neutralized to pH 7 with an 18 wt. % NaOH solution. The viscosity can range from about 1 to about 500 mPa·s in another aspect, from about 10 to about 250 mPa·s in a further aspect, and from about 15 to about 150 mPa·s in a still further aspect.

The low molecular weight HMP may be a non-crosslinked, linear acrylic copolymer having a number average molecular weight of about 15,000 to about 40,000 and is derived from methacrylic acid and ethyl acrylate.

The low molecular weight HMP may be potassium acrylates copolymer.

In addition to LIPCAs, i.e. SACs or HMPs, the cleansing composition may optionally comprise additional ingredients typically used in cleansing compositions. A wide variety of additional ingredients conventionally used in compositions for use on skin and hair, at their art-established levels can be included. For example, the cleansing composition may comprise other polymeric surfactants or monomeric surfactants. Anionic, nonionic, amphoteric, cationic surfactants or mixtures of the foregoing may be used as known in the art. Other optional ingredients include pearlescent or opacifying agents, thickeners, emollients, secondary conditioners, humectants, chelating agents, exfoliants, and additives that

enhance the appearance, feel, or fragrance of the cleansing composition, such as colorants, fragrances, preservatives, pH adjusting agents, and the like.

The cleansing composition may comprise about 0.1 to about 10, preferably from about 0.25 to about 5, percent by weight SAC. The cleansing composition may comprise about 0.5 to about 2.5 percent by weight SAC. The cleansing composition may comprise about 1 to about 2.5 percent by weight SAC.

The cleansing composition may comprise about 0.1 to about 5, preferably from about 0.1 to about 2.5, percent by weight HMP. The cleansing composition may comprise about 0.5 to about 2.5 percent by weight HMP. The cleansing composition may comprise about 1 to about 2.5 percent by weight HMP.

The cleansing composition may be NEUTROGENA® Ultra Gentle Daily Cleanser, commercially available from Johnson & Johnson Consumer Inc.

The leave-on composition comprises at least one retinoid. As used herein, "retinoid" means a compound structurally similar to Vitamin A, such as those characterized by this structure:

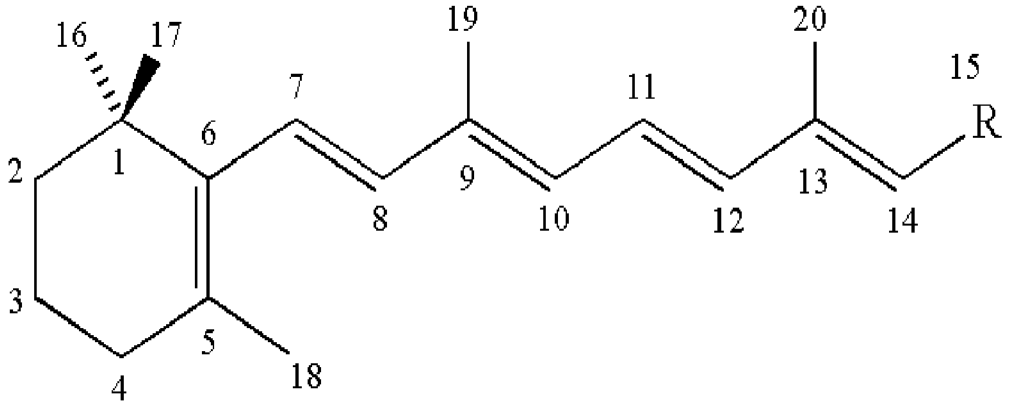

wherein R represents a functional group such as $CH_2OH$ (retinol), CHO (retinal), $CO_2H$ (retinoic acid), $CH_2OCOCH_3$ (retinyl acetate). Retinoids include other esters such as retinyl palmitate, amine derivatives, and the like. The retinoid may be selected from retinol, retinal, retinoic acid, retinyl acetate, and retinyl palmitate. Preferably, the retinoid is retinol.

The leave-on composition may also optionally contain additional ingredients typically used in leave-on compositions. A wide variety of additional oil-soluble materials and/or water-soluble materials conventionally used in compositions for use on skin and hair, at their art-established levels can be included. For example surfactants or emulsifers, pearlescent or opacifying agents, thickeners, emollients, conditioners, humectants, chelating agents, exfoliants, and additives that enhance the appearance, feel, or fragrance of the cleansing composition, such as colorants, fragrances, preservatives, pH adjusting agents, and the like, can be included.

The leave-on composition may comprise at least one polar emollient having a net relative polarity index to the retinoid from about 0.5 to 2, and at least one non-polar emollient having a net relative polarity index to the retinoid from about 7 to about 10, wherein the weight ratio of said polar emollient to said non-polar emollient is from about 95 to 5 to about 40 to 60. For example, the non-polar emollient may be selected from the group consisting of aromatic or linear esters, guerbet ester, mineral oil, squalane, isohexadecane, squalene, liquid paraffin, and mixtures thereof, in particular isohexadecane. The polar emollient may be selected from the group consisting of, propylene glycol stearyl ether, propylene glycol isostearate, and mixtures thereof, in particular propylene glycol stearyl ether.

The leave-on composition may comprise an oil-in-water emulsion comprising: (i) about 0.05 to about 0.5 % by weight retinol; (ii) propylene glycol stearyl ether; and (iii) isohexadecane, wherein the weight ratio of propylene glycol stearyl ether to isohexadecane is from about 75:25 to about 50:50, and the composition does not contain any additional active agents other than retinol.

The leave-on composition may comprise about 0.001 to about 5 percent by weight retinoid. The leave-on composition may comprise about 0.04 to about 2 percent by weight retinoid. The leave-on composition may comprise about 0.04 to about 0.15 percent by weight retinoid.

The leave-on composition may be NEUTROGENA® Rapid Wrinkle Repair®, commercially available from Johnson & Johnson Consumer Inc.

Optional Ingredients, Additional Compositions, and Product Forms

Either or both the cleansing composition and the leave-on composition may comprise other cosmetically acceptable excipients and/or active agents.

Moreover, the present methods, regimens and kits may be optionally practiced using the cleansing composition

and leave-on composition with one or more additional compositions, for example moisturizers, sunscreens, and the like.

Other cosmetically acceptable active agents for use in the cleansing composition, leave-on composition, or additional composition include for example anti-acne agents, shine control agents, anti-microbial agents, anti-inflammatory agents, anti-mycotic agents, anti-parasite agents, external analgesics, sunscreens, photoprotectors, antioxidants, keratolytic agents, surfactants, moisturizers, nutrients, vitamins, energy enhancers, anti-perspiration agents, astringents, deodorants, firming agents, anti-callous agents, agents for hair and/or skin conditioning, and other glycosaminoglycans such as hyaluronic acid.

The amount of cosmetically active agent may range from about 0.001% to about 20% by weight of the composition, e.g., about 0.005% to about 10% by weight of the composition, such as about 0.01% to about 5% by weight of the composition.

The cosmetically acceptable active agent may be selected for instance from hydroxy acids, benzoyl peroxide, D-panthenol carotenoids, ceramides, polyunsaturated fatty acids, essential fatty acids, enzymes, such as laccase, enzyme inhibitors, minerals, hormones, such as estrogens, steroids, such as hydrocortisone, 2-dimethylaminoethanol, copper salts, such as copper chloride, peptides, like argireline, syn-ake, and those containing copper, coenzyme Q10, amino acids, such as proline, vitamins, lactobionic acid, acetyl-coenzyme A, niacin, riboflavin, thiamin, ribose, electron transporters, such as NADH and FADH2, natural extracts, such as those from aloe vera, feverfew, oatmeal, dill, blackberry, princess tree, picia anomala, and chicory, resorcinols, such as 4-hexyl resorcinol, curcuminoids, sugar amines, such as N-acetyl glucosamines, and derivatives and mixtures thereof.

Examples of vitamins include, but are not limited to, vitamin A, vitamin B's, such as vitamin B3, vitamin B5, and vitamin B12, vitamin C, vitamin K, and different forms of vitamin E, like alpha, beta, gamma, or delta tocopherols, or their mixtures, and derivatives thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants, such as sulfhydryl compounds and their derivatives (e.g., sodium metabisulfite and N-acetyl-cysteine), lipoic acid, and dihydrolipoic acid, resveratrol, lactoferrin, and ascorbic acid, and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids, and their derivatives (e.g., genistein and diadzein), extracts containing resveratrol, and the like. Examples of such natural extracts include grape seed, green tea, pine bark, and propolis.

The compositions may further include cosmetically acceptable topical carriers. The carrier may be from about 50% to about 99.99%, by weight, of the composition (e.g., from about 80% to about 99%, by weight, of the composition). Preferably, in the composition of the invention, the cosmetically acceptable topical carrier includes water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up, such as foundations and mascaras. These product types may contain a variety of cosmetically acceptable topical carriers including, but not limited to solutions, suspensions, emulsions, including microemulsions and nanoemulsions, gels, solids, and liposomes. The following are non-limiting examples of such carriers. Other carriers can be formulated by those of ordinary skill in the art.

The compositions can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol, polypropylene glycol, glycerol, 1,2,4-butanetriol, sorbitol esters, 1,2,6-hexanetriol, ethanol, and mixtures thereof.

Compositions may be formulated as a solution comprising an emollient. Such compositions preferably contain from about 2% to about 50% of an emollient(s). As used herein, "emollients" refer to materials used for the prevention or relief of dryness, such as by preventing the transepidermal loss of water from the skin. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook"). Examples of particularly suitable emollients include vegetable oils, mineral oils, fatty esters, and the like.

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

The composition may alternatively be anhydrous or be an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 2979-84.

The compositions may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions can also be formulated as gels (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contain between about 0.1% and 5%, by weight, of such gelling agents.

The compositions can also be formulated into solid formulations (e.g., wax-based sticks, bars, or powders). They may be loaded onto substrates, such as woven or non-woven materials, wipes, patches, masks, articles of clothing and the like.

Retinoid Activity

According to the invention, pretreatment of skin with the cleansing composition comprising a LIPCA provides an increase in the activity of a retinoid thereafter topically applied.

The retinoid may be retinol, and such pretreatment provides at least about a 150%, preferably at least about 180%, increase in retinol bioactivity as measured by a change in cellular retinoic acid binding protein II (CRABPII) gene expression versus treatment with retinol alone. The change in cellular retinoic acid binding protein II (CRABPII) gene expression is measured using the CRABPII EXPRESSSION TEST set forth in Example 1.

The following examples further illustrate the claimed invention.

**Example 1**

The activity of a leave-on retinol product (NEUTROGENA® Rapid Wrinkle Repair® ("RWR")) was tested when combined in a regimen with various mild cleansers. Retinol activity was evaluated using the *in vitro* CRABPII EXPRESSION TEST for Cellular Retinoic Acid Binding Protein II ("CRABPII") expression as follows.

One centimeter-diameter *ex vivo* skin explants were prepared from skin abdominal biopsies. The skin explants were maintained in KGM gold™ culture medium supplemented with amphotericin B, 0.125 μg/ml at 37° C, in a water saturated atmosphere for the 48 hour duration of the test. The explants were placed in a conventional test plate, epidermal surface oriented up, and in sufficient culture medium to nearly but not completely immerse the sample (i.e., the epidermal surface protrudes from the upper surface of the medium). The mild cleanser being tested was applied to the epidermal surface protruding from the culture medium for 4 hours, followed by washing with phosphate buffered saline (PBS) and application of the RWR. After 48 hours, using conventional techniques known to those skilled in the art, the explants are removed, from which epidermal mRNA was extracted and expression of CRABPII gene was measured by Quantitative real time PCR (QRT-PCR) using a suitable sequence of oligonucleotides.

The mild cleansers tested are described in Tables 1 and 2 below. Results are shown as fold-changes from untreated control.

**Table 1**

| Treatment | Fold-change from Untreated (CRABPII gene expression) | Std. deviation |
|---|---|---|
| Untreated | 1 | 0.15 |

(continued)

| Treatment | Fold-change from Untreated (CRABPII gene expression) | Std. deviation |
|---|---|---|
| Cleansing Composition A[1] | 0.85 | 0.05 |
| Comparative Cleansing Composition B[2] | 0.74 | 0.23 |
| RWR | 3.1 | 0.79 |
| Cleansing Composition A + RWR | 5.8 | 0.71 |
| Comparative Cleansing Composition B + RWR | 2.6 | 0.23 |

[1]NEUTROGENA® Ultra Gentle Daily Cleanser commercially available from Johnson & Johnson Consumer Inc. containing : Water, Glycerin, Cocamidopropyl Betaine, Lauryl Glucoside Potassium Acrylates Copolymer, PEG-120 Methyl Dioleate, Disodium Lauroamphodiacetate, Sodium Cocoyl Sarcosinate, Ethylhexylglycerin, Caprylyl, Potassium Sorbate, Fragrance.
[2]OLAY® Sensitive Foaming Face Wash, commercially available from Procter & Gamble containing: Water/Eau, Glycerin, Sodium Myristoyl Sarcosinate, PEG-120 Methyl Glucose Dioleate, Sodium Lauroamphoacetate, Aloe Barbadensis Leaf Juice, Polyquaternium-10, PEG-150 Pentaerythrityl Tetrastearate, Glycol Distearate, Sodium Laureth Sulfate, Cocamide MEA, Laureth-10, Disodium Lauroamphodiacetate, Sodium Trideceth Sulfate, Citric Acid, Disodium EDTA, Phenoxyethanol, DMDM Hydantoin.

**Table 2**

| Treatment | Fold-change from Untreated (CRABPII gene expression) | Std. deviation |
|---|---|---|
| Untreated | 1 | 0.18 |
| RWR | 3.7 | 1.36 |
| Cleansing Composition A[1] + RWR | 7.2 | 2.9 |
| Comparative Cleansing Composition C[2] + RWR | 3.2 | 1.6 |
| Comparative Cleansing Composition D[3] + RWR | 4.3 | 1.3 |

[1]NEUTROGENA® Ultra Gentle Daily Cleanser commercially available from Johnson & Johnson Consumer Inc. containing : Water, Glycerin, Cocamidopropyl Betaine, Lauryl Glucoside Potassium Acrylates Copolymer, PEG-120 Methyl Dioleate, Disodium Lauroamphodiacetate, Sodium Cocoyl Sarcosinate, Ethylhexylglycerin, Caprylyl, Potassium Sorbate, Fragrance.
[2]CETAPHIL Gentle Skin Cleanser, commercially available from Galderma containing: Water, Cetyl Alcohol, Propylene Glycol, Sodium Lauryl Sulfate, Stearyl Alcohol, Methylparaben, Propylparaben, Butylparaben.
[3]CETAPHIL Daily Foaming Cleanser, commercially available from Galderma containing: Water (Purified), Sodium Lauroyl Sarcosinate, Acrylates/Steareth 20 Methacrylate Crosspolymer, Glycerin, PEG 200 Hydrogenated Glyceryl Palmate, Sodium Laureth Sulfate, Butylene Glycol, PEG 7 Glyceryl Cocoate, Phenoxyethanol, Masking Fragrance (Parfum), Panthenol, PEG 60 Hydrogenated Castor Oil, Disodium EDTA, Methylparaben.

These results show pre-treatment of tissues with a cleansing composition comprising a LIPCA according to the invention before application of a retinol product led to an increase in the downstream retinol bioactivity as compared to the retinol product alone, and as compared to pretreatment with cleansing compositions not containing LIPCAs.

**Example 2**

The in-vitro mildness (IL-1$\alpha$ levels) of different treatments was tested as follows.

Epidermal equivalents (EPI 200 HCF), multilayer and differentiated epidermis consisting of normal human

epidermal keratinocytes, were obtained from MatTek (Ashland, MA). On receipt, the epidermal equivalents were incubated for 24 hours at 37°C in maintenance medium without hydrocortisone. Equivalents were topically treated with the indicated products in Table 3 (6$\mu$L volume) for 1 hour, then washed twice with PBS (phosphate buffered saline). Equivalents were then incubated for 24 hours at 37°C with maintenance medium then supernatants were analyzed for IL-1$\alpha$ cytokine release using commercially available kits (Millipore Corp., Billerica, MA).

**Table 3**

| **Treatment** | **Mean** of **IL-1$\alpha$ Release (pg/mL)** |
|---|---|
| Untreated | 77.9 |
| Cleansing Composition A[1] | 243.9 |
| Comparative Cleansing Composition B[2] | 888.3 |
| Comparative Cleansing Composition C[3] | 906.3 |
| Comparative Cleansing Composition D[4] | 91.3 |

[1]NEUTROGENA® Ultra Gentle Daily Cleanser commercially available from Johnson & Johnson Consumer Inc. containing : Water, Glycerin, Cocamidopropyl Betaine, Lauryl Glucoside Potassium Acrylates Copolymer, PEG-120 Methyl Dioleate, Disodium Lauroamphodiacetate, Sodium Cocoyl Sarcosinate, Ethylhexylglycerin, Caprylyl, Potassium Sorbate, Fragrance.
[2]OLAY® Sensitive Foaming Face Wash, commercially available from Procter & Gamble containing: Water/Eau, Glycerin, Sodium Myristoyl Sarcosinate, PEG-120 Methyl Glucose Dioleate, Sodium Lauroamphoacetate, Aloe Barbadensis Leaf Juice, Polyquaternium-10, PEG-150 Pentaerythrityl Tetrastearate, Glycol Distearate, Sodium Laureth Sulfate, Cocamide MEA, Laureth-10, Disodium Lauroamphodiacetate, Sodium Trideceth Sulfate, Citric Acid, Disodium EDTA, Phenoxyethanol, DMDM Hydantoin.
[3]CETAPHIL Daily Foaming Cleanser, commercially available from Galderma containing: Water (Purified), Sodium Lauroyl Sarcosinate, Acrylates/Steareth 20 Methacrylate Crosspolymer, Glycerin, PEG 200 Hydrogenated Glyceryl Palmate, Sodium Laureth Sulfate, Butylene Glycol, PEG 7 Glyceryl Cocoate, Phenoxyethanol, Masking Fragrance (Parfum), Panthenol, PEG 60 Hydrogenated Castor Oil, Disodium EDTA, Methylparaben
[4]CETAPHIL Gentle Skin Cleanser, commercially available from Galderma containing: Water, Cetyl Alcohol, Propylene Glycol, Sodium Lauryl Sulfate, Stearyl Alcohol, Methylparaben, Propylparaben, Butylparaben

These results show that both Cleansing Composition A and Comparative Cleansing Composition D provided low levels of IL-1$\alpha$ release, indicating mildness. However, as shown in Example 1, only Cleansing Composition A containing a LIPCA according to the invention provides both mildness and a boost in retinol activity.

A non-exhaustive list of aspects of the disclosure is set out in the following numbered clauses:

Clause 1. A method for treating skin, comprising in sequence:

(a) cleansing skin in need of treatment for signs of skin aging, acne, or rosacea with a cleansing composition comprising a superhydrophilic amphiphilic copolymer; and

(b) topically applying to the skin a leave-on composition comprising a retinoid.

Clause 2. The method of clause 1, wherein the superhydrophilic amphiphilic copolymer is a starch-based polysaccharide derived from potato or tapioca.

Clause 3. The method of clause 1, wherein the superhydrophilic amphiphilic copolymer is sodium potato dextrin dodecenylsuccinate.

Clause 4. The method of clause 1, wherein the retinoid is selected from the group consisting of retinol, retinal, retinoic acid, retinyl acetate, and retinyl palmitate.

Clause 5. The method of clause 1, wherein the retinoid is retinol.

Clause 6. A kit comprising:

(a) a cleansing composition comprising a superhydrophilic amphiphilic copolymer; and

(b) a leave-on composition comprising a retinoid.

Clause 7. A method of increasing the activity of a retinoid, comprising cleansing skin in need of treatment with retinoids with a cleansing composition comprising a superhydrophilic amphiphilic copolymer prior to contacting the skin with the retinoid.

Clause 8. The method of clause 7, wherein the activity of the retinoid is for treating signs of skin aging.

Clause 9. The method of clause 7, wherein the activity of the retinoid is for treating signs of acne.

Clause 10. The method of clause 7, wherein the activity of the retinoid is for treating signs of rosacea.

Clause 11. A method for treating skin, comprising in sequence:

(a) cleansing skin in need of treatment for signs of skin aging, acne, or rosacea with a cleansing composition comprising a low molecular weight hydrophobically modified polymer; and

(b) topically applying to the skin a leave-on composition comprising a retinoid.

Clause 12. The method of clause 11, wherein the low molecular weight hydrophobically modified polymer is a non-crosslinked, linear acrylic copolymer.

Clause 13. The method of clause 11, wherein the low molecular weight hydrophobically modified polymer is a non-crosslinked, linear acrylic copolymer derived from at least one first monomeric component selected from the group consisting of (meth)acrylic acid and at least one second monomeric component selected from the group consisting of one or more $C_1$ to $C_9$ alkyl (meth)acrylates, wherein the low molecular weight hydrophobically modified polymer has a number average molecular weight of about 100,000 or less.

Clause 14. The method of clause 11, wherein the low molecular weight hydrophobically modified polymer is potassium acrylates copolymer.

Clause 15. The method of clause 11, wherein the retinoid is selected from the group consisting of retinol, retinal, retinoic acid, retinyl acetate, and retinyl palmitate.

Clause 16. The method of clause 11, wherein the retinoid is retinol.

Clause 17. A kit comprising:

(a) a cleansing composition comprising a low molecular weight hydrophobically modified polymer; and

(b) a leave-on composition comprising a retinoid.

Clause 18. A method of increasing the activity of a retinoid, comprising cleansing skin in need of treatment with retinoids with a cleansing composition comprising a low molecular weight hydrophobically modified polymer prior to contacting the skin with the retinoid.

Clause 19. The method of clause 18, wherein the activity of the retinoid is for treating signs of skin aging.

Clause 20. The method of clause 18, wherein the activity of the retinoid is for treating signs of acne.

Clause 21. The method of clause 18, wherein the activity of the retinoid is for treating signs of rosacea.

## Claims

**1.** A leave-on composition comprising a retinoid for use in a method for treating skin showing signs of aging, acne

and/or rosacea wherein the method comprises:

(a) cleansing skin in need of treatment with a cleansing composition comprising a superhydrophilic amphiphilic copolymer; and
(b) topically applying to the skin the leave-on composition comprising a retinoid.

**2.** The composition of claim 1, wherein the superhydrophilic amphiphilic copolymer is a starch-based polysaccharide derived from potato or tapioca.

**3.** The composition of claim 2, wherein the superhydrophilic amphiphilic copolymer is sodium potato dextrin dodecenylsuccinate.

**4.** A kit comprising:

(a) a cleansing composition comprising a superhydrophilic amphiphilic copolymer; and
(b) a leave-on composition comprising a retinoid.

**5.** A retinoid for use in a method for treating skin in need of treatment with retinoids, wherein the method comprises cleansing skin in need of treatment with a cleansing composition comprising a superhydrophilic amphiphilic copolymer prior to contacting the skin with the retinoid, wherein the method increases the activity of the retinoid.

**6.** A leave-on composition comprising a retinoid for use in a method for treating skin showing signs of aging, acne, and/or rosacea wherein the method comprises:

(a) cleansing skin in need of treatment with a cleansing composition comprising a low molecular weight hydrophobically modified polymer; and
(b) topically applying to the skin the leave-on composition comprising a retinoid.

**7.** The composition of claim 6, wherein the low molecular weight hydrophobically modified polymer is a non-crosslinked, linear acrylic copolymer.

**8.** The composition of claim 7, wherein the low molecular weight hydrophobically modified polymer is a non-crosslinked, linear acrylic copolymer derived from at least one first monomeric component selected from the group consisting of (meth)acrylic acid and at least one second monomeric component selected from the group consisting of one or more $C_1$ to $C_9$ alkyl (meth)acrylates, wherein the low molecular weight hydrophobically modified polymer has a number average molecular weight of about 100,000 or less.

**9.** The composition of claim 8, wherein the low molecular weight hydrophobically modified polymer is potassium acrylates copolymer.

**10.** The composition of any one of claims -1-3 or 6-9, wherein the retinoid is selected from the group consisting of retinol, retinal, retinoic acid, retinyl acetate, and retinyl palmitate.

**11.** The composition of claim 10, wherein the retinoid is retinol.

**12.** A kit comprising:

(a) a cleansing composition comprising a low molecular weight hydrophobically modified polymer; and
(b) a leave-on composition comprising a retinoid.

**13.** A retinoid for use in a method for treating skin in need of treatment with retinoids, wherein the method comprises cleansing skin in need of treatment with a cleansing composition comprising a low molecular weight hydrophobically modified polymer prior to contacting the skin with the retinoid, wherein the method increases the activity of the retinoid.

**14.** The retinoid of claims 5 or 13 for use in a method for treating skin showing signs of:

(i) skin aging,
(ii) acne, and/or

(iii) rosacea.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010085753 A
- US 8258250 B
- US 2661349 A
- US 5954883 A
- US 8025902 B
- US 6433061 B, Marchant


### Non-patent literature cited in the description


- International Cosmetic Ingredient Dictionary and Handbook. Personal Care Products Council
- International Cosmetic Ingredient Dictionary and Handbook. The Cosmetic, Toiletry, and Fragrance Assoc, 2002, 2930-36
- ICI Handbook. 2979-84
- ICI Handbook. 2962-71